# EUROPEAN PATENT APPLICATION

(11) **EP 4 810 076 A2**
(43) Date of publication of application: **23.09.2026**
(21) Application number: 26154918.2
(22) Date of filing: 29.01.2026
(51) Int. Cl.: A61M 1/36, A61M 1/16, A61M 60/38, A61M 60/109, A61M 60/113, A61M 60/279, A61M 60/554

(54) **HEART LUNG MACHINE WITH ROLLER PUMP**

(30) Priority: 18.03.2025 US 202519082934
(71) Applicant: LivaNova Deutschland GmbH, 80939 Munich (DE)
(72) Inventor: Schubert, Friedemann, 80639 München (DE); Michel, Günther Ludwig, 80939 München (DE); Löffler, Philipp, 85356 Freising (DE)
(74) Representative: Grünecker Patent- und Rechtsanwälte PartG mbB

(57) **Abstract**

Example extracorporeal life support systems are disclosed. An example heart lung machine includes a roller pump configured to pump blood through a blood circuit, a first pump tube engaged with a portion of the roller pump, a first connector having a first end and a second end, wherein the first end of the first connector is coupled to a first end of the pump tube. The heart lung machine also includes a first pressure sensor coupled to the first connector or the roller pump and a sensor interface unit coupled to the roller pump, wherein the sensor interface unit is in communication with the first pressure sensor.

## Description

### TECHNICAL FIELD

The present disclosure relates to an extracorporeal life support system and methods for manufacturing and/or using an extracorporeal life support system. More particularly, the present disclosure relates to components for extracorporeal life support systems, in particular, for heart lung machines.

### BACKGROUND

Some medical procedures (e.g., medical procedures which treat cardiac or respiratory disease) may require the use of a life support system that supports cardiac and pulmonary functions by artificially supporting the heart and the lung function. In some instances, this may be carried out by a heart lung machine. A heart lung machine may provide both cardiac and respiratory support to a patient whose heart and lungs are unable to provide an adequate amount of gas exchange during a cardiac and pulmonary procedure. Heart lung machines work by removing blood from a patient's body to oxygenate the red blood cells while also removing carbon dioxide. The oxygenated blood is then returned to the patient.

Heart lung machines may include multiple devices that together form a blood recirculation loop between a patient and a blood oxygenator. For example, some heart lung machines may include a blood reservoir, a blood pump to power blood flow, an oxygenator to oxygenate the blood, a device to filter the blood (which may be included within the oxygenator and/or the reservoir in some systems), one or more sensors positioned at various locations along blood pathways and one or more control units. It can be appreciated that a blood pathway (e.g., tubing) may extend from the patient to the blood reservoir, then towards a blood pump, then pass through the oxygenator and close the loop by returning to the patient. Accordingly, the blood pump may assist the heart by pumping blood through the circulation loop, while the oxygenator may assist the lungs by oxygenating blood that is eventually returned to the patient.

Heart lung machines may often include a roller pump that uses rotating rollers to compress blood tubing, effectively creating a peristaltic motion that creates positive-pressure blood flow through the blood circuit by squeezing it along blood tubing. Further, when incorporating a roller pump, heart lung machines often also include one or more pressure sensors configured to provide a safety feature by monitoring the pressure within the blood circuit, allowing the system to detect potential blockages or excessive pressures. In some examples, the pressure sensors may be positioned away from the blood pump, thereby requiring a length of tubing to extend from the blood pump to the pressure sensors. Accordingly, for ease and efficiency of use, it may be beneficial to position the pressure sensors adjacent the blood pump, thereby reducing the length of tubing, cables and/or number of connectors during set-up and operation the heart lung machine.

### BRIEF SUMMARY

This disclosure provides design, material, manufacturing method, and use alternatives for medical devices and/or systems, namely circulatory support devices, including heart lung machines, components thereof, and associated devices. An example heart lung machine includes a roller pump configured to pump blood through a blood circuit, a first pump tube engaged with a portion of the roller pump, a first connector having a first end and a second end, wherein the first end of the first connector is coupled to a first end of the pump tube. The heart lung machine also includes a first pressure sensor coupled to the first connector or the roller pump and a sensor interface unit coupled to the roller pump, wherein the sensor interface unit is in communication with the first pressure sensor.

Alternatively or additionally to any of the embodiments above, wherein the first pressure sensor is attached to the first connector.

Alternatively or additionally to any of the embodiments above, wherein the second end of the first connector is attached to a fluid tube of the blood circuit.

Alternatively or additionally to any of the embodiments above, wherein the first connector includes an inner surface defining a lumen, and wherein the first pressure sensor is configured to be positioned within a recess extending along the inner surface of the first connector.

Alternatively or additionally to any of the embodiments above, wherein the first pressure sensor is configured to directly contact fluid flowing through the lumen.

Alternatively or additionally to any of the embodiments above, further comprising an electrical connection extending between the first pressure sensor and the sensor interface unit.

Alternatively or additionally to any of the embodiments above, wherein the first end of the first connector is releasably attached to a retention member of the pump.

Alternatively or additionally to any of the embodiments above, wherein the first end of the first connector extends through a wall of the retention member of the pump.

Alternatively or additionally to any of the embodiments above, wherein a second end of the pump tube is coupled to a first end of a second connector, wherein the first end of the second connector extends through a wall of the retention member of the pump, and wherein the entire pump tube is positioned within the pump.

Alternatively or additionally to any of the embodiments above, wherein a first end of the fluid tube is configured to extend into a lumen defined by an inner surface of the first connector, and wherein the first pressure sensor is configured to be positioned between an outer surface of the fluid tube and the inner surface of the first connector.

Alternatively or additionally to any of the embodiments above, wherein the first pressure sensor is configured to be positioned within a recess extending along the outer surface of the fluid tube.

Alternatively or additionally to any of the embodiments above, wherein a wall of the fluid tube separates the first pressure sensor from a fluid flowing through a lumen of the fluid tube.

Alternatively or additionally to any of the embodiments above, wherein the wall of the fluid tube is configured to engage the first pressure sensor in response to a change in a pressure of the fluid flowing through the lumen of the fluid tube.

Another example heart lung machine includes a roller pump configured to pump blood through a blood circuit. The heart lung machine also includes a connector assembly coupled to the roller pump. The connector includes a connector having a first end region, a second end region and an inner surface and a pressure sensor attached to the inner surface. Further, the first end of the connector is releasably engaged to the roller pump and the second end of the connector is engaged to a fluid tube of the blood circuit.

Alternatively or additionally to any of the embodiments above, wherein the pressure sensor is configured to be positioned within a recess extending along the inner surface of the connector.

Alternatively or additionally to any of the embodiments above, wherein the pressure sensor is configured to directly contact fluid flowing through a lumen defined by the inner surface of the connector.

Alternatively or additionally to any of the embodiments above, further comprising an electrical connection extending between the pressure sensor and a sensor interface unit positioned within the roller pump.

Alternatively or additionally to any of the embodiments above, wherein a first end of the fluid tube is configured to extend into a lumen defined by the inner surface of the connector, and wherein the pressure sensor is positioned between an outer surface of the fluid tube and the inner surface of the connector.

Alternatively or additionally to any of the embodiments above, wherein the pressure sensor is configured to be positioned within a recess extending along the outer surface of the fluid tube.

Another example heart lung machine includes a roller pump configured to pump blood through a blood circuit, a first pump tube releasably engaged with a portion of the roller pump a first connector having a first end, a second end and a first sensor manifold, wherein the first end of the first connector is coupled to a first end of the pump tube, wherein the second end of the first connector is coupled to a fluid tube of the blood circuit, and wherein the sensor manifold is coupled to a first pressure sensor. Further, the heart lung machine also includes a second connector having a first end, a second end and a second sensor manifold, wherein the first end of the second connector is coupled to a second end of the pump tube, wherein the second end of the second connector is coupled to a fluid tube of the blood circuit, and wherein the second sensor manifold is coupled to a second pressure sensor. Further, the heart lung machine also includes a sensor interface unit positioned within the roller pump, wherein the first pressure sensor, the second pressure sensor or both the first and the second pressure sensor are directly attached to the roller pump, and wherein the sensor interface unit is in communication with the first pressure sensor and the second pressure sensor.

The above summary of some embodiments is not intended to describe each disclosed embodiment or every implementation of the present disclosure. The Figures, and Detailed Description, which follow, more particularly exemplify these embodiments.

### BRIEF DESCRIPTION OF THE DRAWINGS

The disclosure may be more completely understood in consideration of the following detailed description in connection with the accompanying drawings, in which:
FIG. 1 illustrates an example heart lung machine;
FIG. 2 illustrates a portion of the heart lung machine of FIG. 1;
FIG. 3 illustrates a portion of a pump of the heart lung machine of FIG. 1;
FIG. 4 illustrates an example pump of a heart lung machine;
FIG. 5 illustrates an example pump of a heart lung machine;
FIG. 6 illustrates a portion of a pump of the heart lung machine of FIG. 5;
FIG. 7 illustrates an example connector of the heart lung machine of FIG. 5;
FIG. 8 illustrates an example connector of the heart lung machine of FIG. 5;
FIG. 9 illustrates a portion of an example pump of a heart lung machine;
FIG. 10 illustrates an example of a portion of the pump of FIG. 9; and
FIG. 11 illustrates another example of a portion of the pump of FIG. 9.

While the disclosure is amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications, equivalents, and alternatives falling within the spirit and scope of the disclosure.

### DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the disclosure.

In a normal heart, blood circulates via a closed path whereby deoxygenated (venous) blood enters the right atrium via both the superior vena cava and inferior vena cava. The venous blood then passes through the right ventricle and is pumped via the pulmonary artery to the lungs, where it absorbs oxygen and releases carbon dioxide. After absorbing oxygen and releasing carbon dioxide in the lungs, the blood becomes oxygenated arterial blood. The oxygenated blood is then returned via the pulmonary veins to the left atrium and is passed to the left ventricle. The oxygenated arterial blood is then pumped through the aorta and eventually throughout the body.

It can be appreciated that if the lungs are incapable of sufficiently oxygenating blood and/or removing carbon dioxide, an oxygenator located outside the body may be used to oxygenate the blood and/or remove carbon dioxide. As discussed above, extracorporeal perfusion via a heart lung machine may be utilized to support patients while medical treatments (e.g., heart surgery) are performed to treat their underlying illness. When supported via a heart lung machine, oxygenation of the patient's blood and removal of carbon dioxide may occur outside the body.

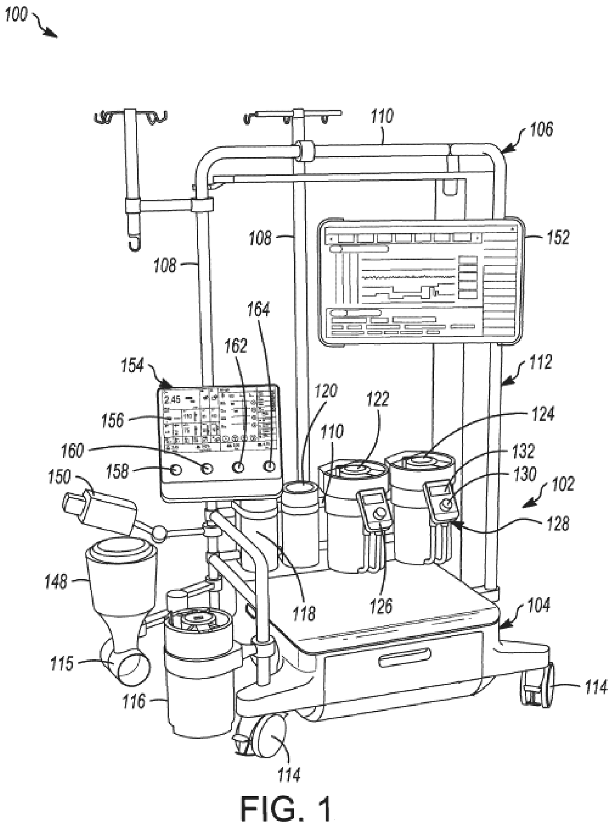

FIG. 1 is a perspective view of an example heart lung machine 100. As shown, the heart lung machine 100 may include a trolley 102 having a base 104 that includes an internal cavity (not shown) for housing any number of different controls, electrical circuits, hydraulic circuits, a battery discharger, and/or the like. For example, in embodiments, a peripheral processing unit may be disposed within the base 104. A mast assembly 106 may be coupled to the base 104 and extends upwards from the base 104. The mast assembly 106 may include any number of different mast components, including vertical poles 108, horizontal rails 110, and/or the like. In embodiments, the trolley 102 may include an enclosure 112 that is configured to facilitate cable management, provide A/C outlets, include a power switch for the heart lung machine 100, include an extension box, and/or the like. As shown, the trolley 102 also may include wheels 114 coupled to the base 104.

As shown, the heart lung machine 100 also may include a number of different types of components such as an oxygenator 115 (which may actually be considered to be an element of an extracorporeal circuit used with the heart lung machine 100, but may be referred to herein as being a component of the heart lung machine due to being connected to the trolley 102) and the pumps 116, 118, 120, 122, 124. In some embodiments, one or more of the components 115, 116, 118, 120, 122, and 124 (and/or other components) may be coupled to any number of different portions of the mast assembly 106, and may include, for example, an exposed actuator control unit. For example, the pumps 122, 124 may each include an exposed actuator control unit 126, 128, operably connected thereto, respectively. As shown, an actuator control unit 128 may include a control knob 130 configured to receive user input (e.g., manipulation of the knob 130) for controlling operation of the pump 124, and an information display device 132 configured to present information associated with the pump such as, for example, one or more parameters (e.g., measured device parameters such as, for instance, flow, rpm, etc.). In some embodiments, an actuator control unit may be configured to facilitate control of a pump, a motorized clamp, a motorized occluder, an infusion device, and/or any number of other types of devices that may be associated with a heart lung machine.

Traditionally, heart lung machines have utilized roller pumps that are each integrated into a modular console component. The modular console components are stacked next to one another on the base of a heart lung machine to provide an array of pumps. The modular console component also houses an actuator control unit having an interface for controlling the corresponding integrated roller pump. One advantage of having the actuator control unit interface provided at the modular console component is that, during an emergency situation, the perfusionist can easily determine the actuator control unit that corresponds to a particular roller pump. More recently, mast mounted roller pumps (without the modular console component housing) have been utilized in heart lung machines. Mast mounted pumps provide more flexibility in the configuration of the heart lung machine, however, if the actuator control units for the mast mounted pumps are located remotely, or detached from, the mast mounted pumps, it is potentially more difficult for the perfusionist to identify the actuator control unit that controls a particular pump.

Embodiments of the present disclosure include mast mounted roller pumps, such as pumps 122,124 of FIG. 1, having corresponding actuator control units, such as the actuator control units 126, 128, respectively. The actuator control units 126, 128 may be attached, connected, or otherwise operatively coupled to the corresponding mast mounted roller pumps 122, 124. The connectedness, or close proximity of the actuator control unit to the mast mounted roller pump allows the user to precisely determine the actuator control unit that controls a particular mast mounted roller pump in a high pressure, or emergency situation, including a situation where the control display device 156 is not functioning properly or is disabled. Thus, the ability to mount the pumps 122, 124 to the mast assembly 106 allows a much wider range of configurations to meet the user's particular needs.

As is further shown in FIG. 1, the heart lung machine 100 may include any number of other components such as, for example, a venous reservoir 148 (which may be, for example, a component of an extracorporeal circuit that may be used in combination with the heart lung machine 100, an electronic venous occluder 150, a peripheral display device 152, a control assembly 154, various types of sensors, etc. In some examples, any number of the components discussed herein, others not discussed herein, or aspects of the components (e.g., sensors and/or actuators associated with components) may be operably connected to the peripheral processing unit, which may be configured to receive parameter data from any one or more of the components, process parameter data, receive control signals from any one or more input device and/or provide control signals to any one or more of the components.

In some examples, the peripheral display device 152 may be operably connected to the peripheral processing unit and configured to present a set of parameter data received from the peripheral processing unit. In some examples, the peripheral display device 152 may be, include, or be included within a data recording and/or management system. For example, the peripheral display device 152 may include, or be otherwise associated with, a processing unit separate from that of the heart lung machine, and/or may be configured to record and/or display any number of different operative heart lung machine parameters. In some implementations, for example, the peripheral display device 152 may be configured to obtain and record all of the operative heart lung machine parameter values and/or patient parameters provided by any number of additional monitoring devices. The peripheral display device 152 may be configured to present, graphically, representations of any number of the obtained parameter values, changes in parameter values over time, derived parameter values (e.g., values derived from parameter values), and/or the like.

During operation, the primary focus of a user of the HLM 100 generally is the oxygenator 115 and the venous reservoir 148. Accordingly, embodiments of the subject matter disclosed herein provide a control assembly 154 near those two components 115 and 148 so that the user can access control devices and view displayed parameters without having to move away from, or be distracted from, the oxygenator 115 and venous reservoir 148. According to embodiments, the control assembly 154 may include a control display device 156 and a number of input control devices 158, 160, 162, and 164.

In some examples, the peripheral display device 152 and/or the control display device 156 may include an input mechanism configured to enable user interaction with one or more features displayed on the display device 152 and/or 156. For example, the peripheral display device 152 and/or the control display device 156 may be, or include, a touchscreen device configured to receive user input. In some examples, the peripheral display device 152 and/or the control display device 156 may include an input device connected thereto such as, for example, a mouse, a trackpad and/or a joystick.

In some examples, additional data from devices external to the heart lung machine (e.g., blood gas monitors, electrocardiographs, ventilators, patient monitors, etc.) may be displayed on the peripheral display device 152. As indicated above, the peripheral display device 152 may be controlled by a peripheral processing unit that is separate from the central system unit, the control display device or any other central unit of the heart lung machine. The peripheral processing unit associated with the peripheral display device 152 may be configured to obtain parameter values (e.g., from the central system unit, sensors, actuators, external devices, etc.) and may be configured to collect the data in a database. The peripheral processing unit may be communicatively coupled to the peripheral display device 152, heart lung machine components, and/or external devices.

The illustrative heart lung machine 100 shown in FIG. 1 is not intended to suggest any limitation as to the scope of use or functionality of embodiments of the present disclosure. The illustrative heart lung machine 100 also should not be interpreted as having any dependency or requirement related to any single component or combination of components illustrated therein. Additionally, various components depicted in FIG. 1 may be integrated with various other components depicted therein (and/or components not illustrated), all of which are considered to be within the scope of the present disclosure.

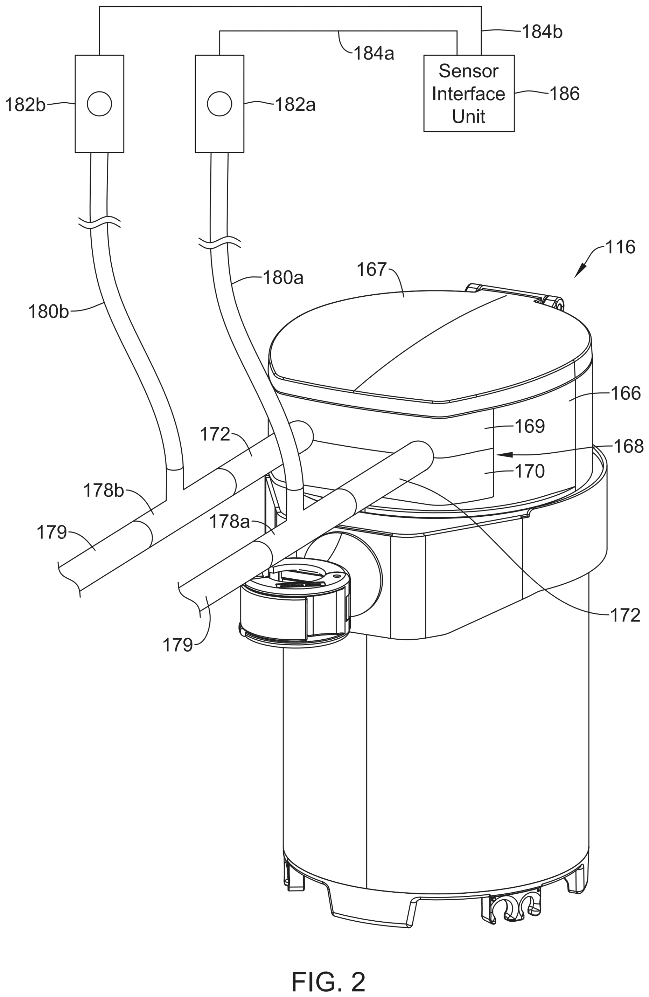

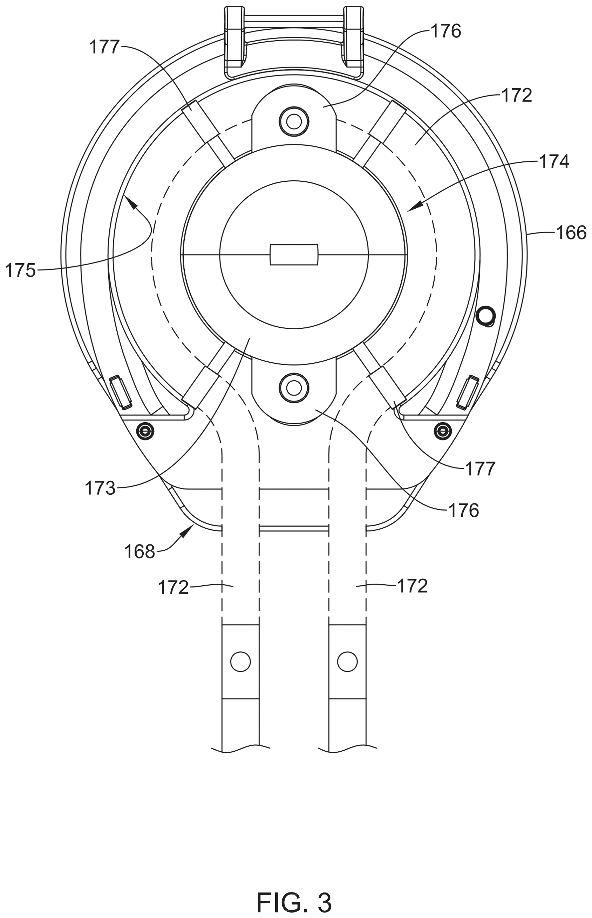

FIGS. 2-3 illustrate the example roller pump 116 of the heart lung machine 100 shown in FIG. 1. FIG. 2 illustrates that the roller pump 116 may include a pump stator 166 and a lid 167 configured to be pivotably disposed over the top of a pump stator 166. FIG. 2 illustrates that the pump stator 166 may include a retention member 168 configured to retain a pump tube 172 (also shown in FIG. 3). In some examples, the retention member 168 may be detachable from the pump stator 166. Further, in some examples, the retention member 168 may include an upper portion 169 and a lower portion 170 that are configured to be brought together to enclose the pump tube 172 within two apertures formed within retention member 168. In other examples, the retention member 168 may be constructed as a monolithic member whereby the pump tube 172 extends through two apertures formed within the wall of the retention member 168.

FIG. 3 illustrates a top view of the illustrative roller pump 116, whereby the lid 167 has been removed. As shown in FIG. 3, the roller pump 116 further includes a pump rotor 173 disposed within the pump stator 166. The pump stator 166 may include a chamber 174 defined therein, and bounded (at least peripherally) by an inner wall 175 of the pump stator 166. The chamber 174 may be approximately cylindrical in shape.

As shown in FIG. 3, the roller pump 116 may be configured such that the pump tube 172 may be inserted into the chamber 174 in such a manner as to contact or lie against the inner wall 175. The inner wall 175 may be referred to, in some examples, as a pump bed. The pump rotor 173 also may include rotatably supported rollers 176 configured to roll along the pump tube 172, thereby compressing the pump tube 172 against the inner wall 175 of the pump stator 166. In response to the compression, the fluid (e.g., blood) present in the pump tube 172 may be transported in the direction of rotation of the pump rotor 173. In some examples, as shown in FIG. 3, the pump rotor 173 may also include one or more roller guides 177 extending radially from the pump rotor 173 and configured to be positioned above and below the pump tube 172, thereby keeping the pump tube 172 aligned to the inner wall 175 of the pump stator 166.

FIG. 3 further illustrates the pump tube 172 extending through two apertures formed within the wall of the retention member 168. Further, it can be appreciated that any number of mechanisms may be used to removably secure the pump tube 172 to the tube retention device 168, including, for example, an interference fit, clips, clamps, corresponding posts and apertures, spring plates, and/or any other suitable fixation mechanism.

As discussed herein, FIG. 2 illustrates that the heart lung machine 100 shown in FIG. 1 may include one or more pressure sensors 182a, 182b in fluid communication with the roller pump 116. It can be appreciated that the pressure sensors 182a, 182b may monitor the pressure within the blood flow circuit of the heart lung machine 100, thereby allowing the system to detect potential blockages or excessive pressures in the blood flow circuit. For example, each of the pressure sensors 182a, 182b may be configured to measure the pressure of a fluid passing through the sensor and convert that pressure into a voltage output. Further, the voltage output may be utilized by a sensor interface unit 186 to detect potential blockages or excessive pressures in the blood flow circuit.

As illustrated in FIG. 2, in some examples, the pressure sensors 182a, 182b may be positioned away from the blood pump, thereby requiring a length of tubing to extend from the blood pump to the pressure sensors. For example, FIG. 2 illustrates that a first end of the pump tube 172 may be connected to a first connector 178a and a second end of the pump tube 172 may be connected to a second connector 178b. It can be appreciated that each of the connectors 178a, 178b may be connected to fluid tubing 179 of the extracorporeal blood flow circuit.

FIG. 2 further illustrates that the connector 178a may be connected to a pressure tube 180a which is configured to extend between the connector 178a and the pressure sensor 182a. Similarly, FIG. 2 illustrates that the connector 178b may be connected to a pressure tube 180b which is configured to extend between the connector 178b and the pressure sensor 182b. As discussed herein, each of the pressure sensors 182a, 182b may monitor pressure within the blood flow circuit of the heart lung machine 100 via fluid passing from the tubing 179 through each of the connectors 178a, 178b and the pressure tubing 180a, 180b to the pressure sensors 182a, 182b, whereby each of the pressure sensors 182a, 182b may measure the pressure of a fluid passing through the sensor and convert that pressure into a voltage output. Further, the voltage output may be sent from the sensor 182a to the sensor interface unit 186 via the electrical connection 184a while the voltage output may be sent from the sensor 182b to the sensor interface unit 186 via the electrical connection 184b. The voltage output may be utilized to detect potential blockages or excessive pressures in the blood flow circuit.

As discussed herein, in some examples, the pressure sensors 182a, 182b may be mounted a distance away from the blood pump 116, thereby requiring the pressure tubes 180a, 180b extending between the blood pump 116 and the pressure sensors 182a, 182b to be relatively long in length. It can be appreciated that the longer length pressure tubes 180a, 180b may interfere with one or more components of the heart lung machine 100. Accordingly, it may be beneficial to configure a heart lung machine in which the pressure sensors 182a, 182b and/or sensor interface unit 186 are mounted adjacent to the pump 116.

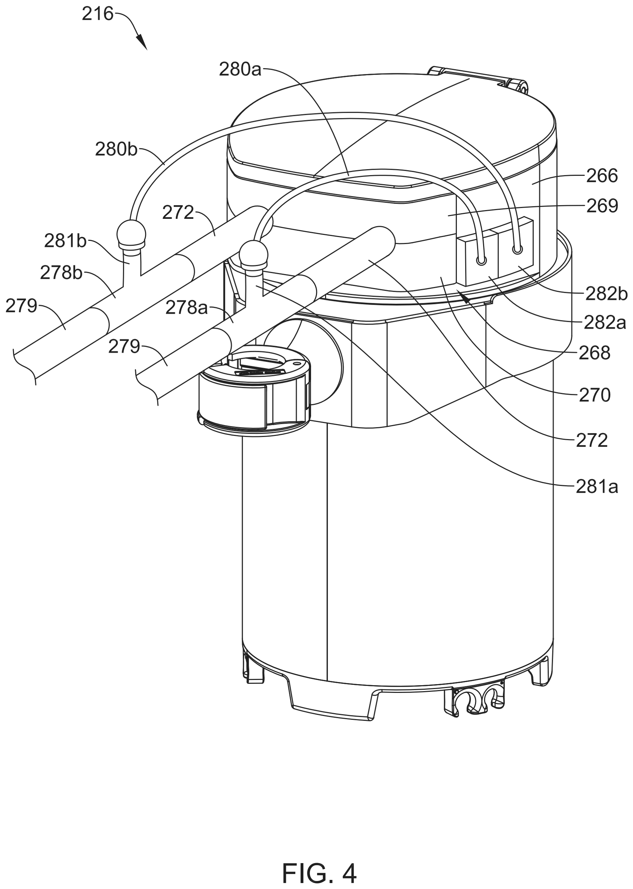

FIG. 4 illustrates another example roller pump 216. The roller pump 216 may be similar in form and function to the roller pump 116 utilized with the heart lung machine 100 described with respect to FIG. 1. For example, FIG. 4 illustrates that the pump 216 may include a pump stator 266 and a retention member 268 configured to retain a pump tube 272 similar in form and function to the stator 166, retention member 168 and pump tube 172 described herein. In some examples, the retention member 268 may be detachable from the pump stator 266. Further, in some examples, the retention member 268 may include an upper portion 269 and a lower portion 270 that are configured to be brought together to enclose the pump tube 272 within two apertures formed within retention member 268. In other examples, the retention member 268 may be constructed as a monolithic member whereby the pump tube 272 extends through two apertures formed within the wall of the retention member 268.

FIG. 4 further illustrates that a first end of the pump tube 272 may be connected to a first connector 278a and a second end of the pump tube 272 may be connected to a second connector 278b. It can be appreciated that each of the connectors 278a, 278b may be connected to tubing 279 of the extracorporeal blood flow circuit.

Additionally, FIG. 4 illustrates that the connector 278a may include a first connector connection (e.g., manifold) 281a connected to a pressure tube 280a, whereby the pressure tube 280a is configured to extend between the connector 278a and a pressure sensor 282a. Similarly, FIG. 4 illustrates that the connector 278b may include a second connector connection (e.g., manifold) 281b connected to a pressure tube 280b, whereby the pressure tube 280b is configured to extend between the connector 278b and the pressure sensor 282b. As discussed herein, each of the pressure sensors 282a, 282b may monitor pressure within the blood flow circuit of the heart lung machine 100 via fluid passing from the tubing 279 through each of the connectors 278a, 278b and the pressure tubing 280a, 280b to the pressure sensors 282a, 282b, whereby each of the pressure sensors 282a, 282b may measure the pressure of a fluid passing through the sensor and convert that pressure into a voltage output. Further, the voltage output may be sent from the sensors 282a, 282b to a sensor interface unit integrated within the pump 216. The voltage output may be utilized to detect potential blockages or excessive pressures in the blood flow circuit.

It can be appreciated from comparing FIG. 2 to FIG. 4 that the length of the pressure tubes 280a, 280b shown in FIG. 4 are shorter than the pressure tubes 180a, 180b shown in FIG. 2. Further, FIG. 4 illustrates that the pressure sensors 282a, 282b may be integrated with the stator 266 of the pump 216. For example, FIG. 4 illustrates the pressure sensors 282a, 282b positioned on an outer surface of the stator 216. In other examples, the pressure sensors 282a, 282b may be positioned inside the pump 216 whereby the pressure tubes 280a, 280b may extend through the wall of the stator 216 to engage the pressure sensors 282a, 282b may be positioned inside the pump 216. It can be appreciated that the integration of the pressure sensors 282a, 282b with the pump 216 together with the relatively short pressure tubes 280a, 280b provides a streamlined, well-organized and efficient arrangement of the connectors 278a, 278b, the pressure tubes 280a, 280b, the pressure sensors 282a, 282b and the pump 216.

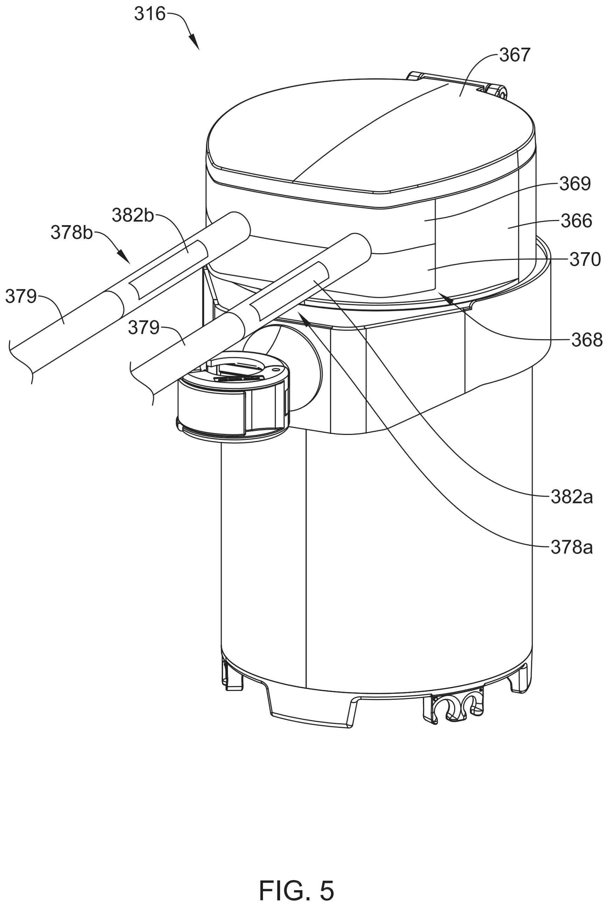

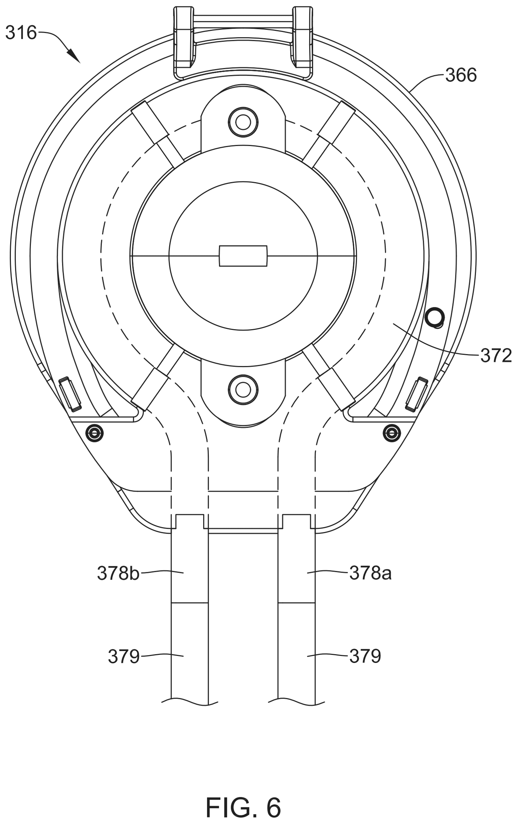

FIGS. 5-6 illustrate another example roller pump 316. The roller pump 316 may be similar in form and function to the roller pump 116 utilized with the heart lung machine 100 described with respect to FIG. 1. For example, FIG. 5 illustrates that the pump 316 may include a pump stator 366 and a retention member 368 configured to retain a pump tube 372 (shown in FIG. 6) similar in form and function to the stator 166, retention member 168 and pump tube 172 described herein. In some examples, the retention member 368 may be detachable from the pump stator 366.

Further, FIG. 6 illustrates a top view of the illustrative roller pump 316, whereby the lid 367 (shown in FIG. 5) has been removed. FIG. 6 illustrates the pump tube 372 positioned within the pump stator 366. FIG. 6 further illustrates that a first end of the pump tube 372 may be connected to a first connector 378a and a second end of the pump tube 372 may be connected to a second connector 378b. It can be appreciated that each of the connectors 378a, 378b may be connected to tubing 379 of the extracorporeal blood flow circuit.

FIGS. 5-6 further illustrate that each of the connectors 378a, 378b may extend through two apertures formed within the wall of the retention member 368. Further, FIG. 5 illustrates that the retention member 368 may include an upper portion 369 and a lower portion 370 that are configured to be brought together to enclose each of the connectors 378a, 378b within two apertures formed within retention member 368. In other examples, the retention member 368 may be constructed as a monolithic member whereby each of the connectors 378a, 378b extends through two apertures formed within the wall of the retention member 368.

Further, it can be appreciated that each of the connectors 378a, 378b may be integrated with the retention member 368. It can be further appreciated that any number of mechanisms may be used to removably secure the connectors 378a, 378b to the tube retention device 368. In some examples, the upper portion 369 and the lower portion 370 may be separated from one another to release each of the connectors 378a, 378b and the pump tube 372 from the stator 366. In other examples, the connectors 378a, 378b and the pump tube 372 may be releasably attached to the retention member 368 via an interference fit, clips, clamps, corresponding posts and apertures, spring plates, and/or any other suitable fixation mechanism. Accordingly, it can be appreciated that the connectors 378a, 378b together with the pump tube 372 may be disposable. In other words, the connectors 378a, 378b together with the pump tube 372 may be removeable from the pump 316, thereby permitting the connectors 378a, 378b and the pump tube 372 to be replaced for each medical procedure in which the heart lung machine 100 is utilized.

FIG. 5 further illustrates that each of the connectors 378a, 378b may be configured to include a pressure sensor. For example, FIG. 5 illustrates a pressure sensor 382a integrated with the connector 378a and a pressure sensor 382a integrated with the connector 378b. It can be appreciated that the pressure sensors 382a, 382b may monitor pressure within the blood flow circuit of the heart lung machine 100 via monitoring fluid passing through each of the connectors 378a, 378b, respectively. The pressure sensors 382a, 382b, integrated with each of the connectors 378a, 378b, respectively, may measure the pressure of a fluid passing through the sensor 382a, 382b and convert that pressure into a voltage output. Further, the voltage output may be sent from the sensors 382a, 382b to a sensor interface unit integrated within the pump 316. The voltage output may be utilized to detect potential blockages or excessive pressures in the blood flow circuit. It can be appreciated that the integration of the pressure sensors 382a, 382b with the pump 316 provides a streamlined, well-organized and efficient arrangement of the connectors 378a, 378b (including the pressure sensors 382a, 382b) and the pump 316.

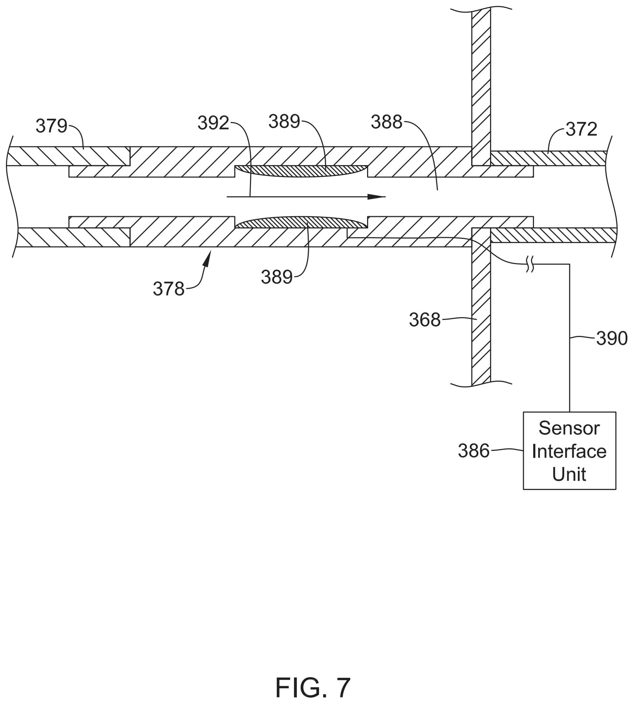

FIG. 7 illustrates an example connector 378 which may be similar in form and function to the connectors 378a, 378b described herein. FIG. 7 illustrates that the connector 378 may include a first end region which is configured to be attached to tubing 379 of the blood flow circuit. As described herein, FIG. 7 further illustrates that the connector 378 may include a second end which is releasably attached to the retention device 368. FIG. 7 further illustrates that the connector 378 may further include a pressure sensor 389 integrated within at least a portion of the wall of the connector 378. For example, the pressure sensor 389 may be positioned within a recess extending along the inner surface of the connector 378. Accordingly, as shown in FIG. 7, the pressure sensor 389 may be in direct contact with fluid passing through an inner lumen 388 of the connector 378. The flow of fluid through the connector 378 is depicted by the arrow 392. It can be appreciated that, in some examples, having the pressure sensor 389 directly contact the fluid passing through the inner lumen 388 of the connector 378 may provide higher accuracy measurements as compared with pressure sensors which are not in direct contact with the fluid passing through the inner lumen 388.

FIG. 7 further illustrates that the connector 378 may further include an electrical connection 390 (e.g., electrical wire, etc.) which may extend from the pressure sensor 389 to a sensor interface unit 390 located within the pump 316 (shown in FIG. 5). As discussed herein, the pressure sensor 382 may measure the pressure of a fluid passing through the lumen 388 of the connector 378 and convert that pressure into a voltage output. Further, the voltage output may be sent from the sensor 389 to the sensor interface unit 386 integrated within the pump 316. The voltage output may be utilized to detect potential blockages or excessive pressures in the blood flow circuit.

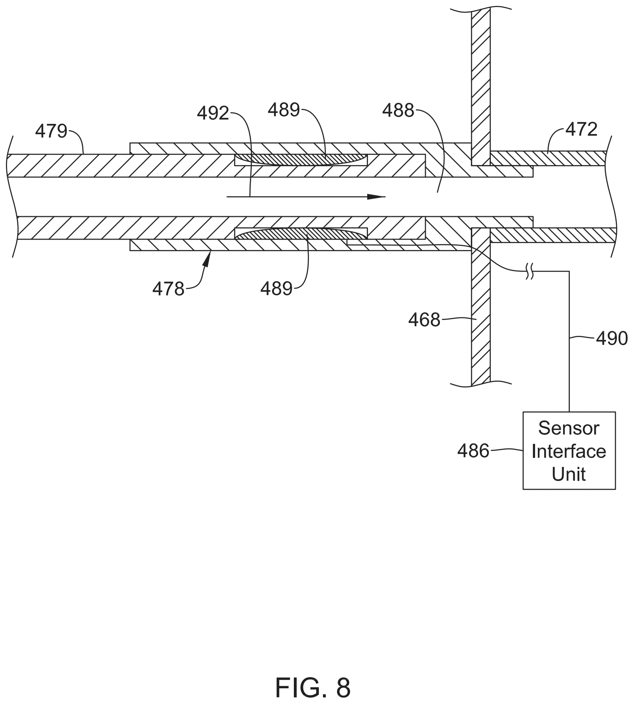

FIG. 8 illustrates another example connector 478 which may be similar in form and function to the connectors 378a, 378b described herein. FIG. 8 illustrates that the connector 478 may include a first end region which is configured to be attached to tubing 479 of the blood flow circuit. FIG. 8 illustrates that a portion of the tubing 479 may extending into a lumen 488 of the connector 478. Further, FIG. 8 further illustrates that the connector 478 may include a second end which is releasably attached to a retention device 468. The retention device 468 may be similar in form and function to the retention device 368 described herein.

FIG. 8 further illustrates that the connector 478 may further include a pressure sensor 489 integrated within at least a portion of the tubing 479 and an inner wall of the connector 478. In other words, FIG. 8 illustrates that the pressure sensor 489 may be positioned between an outer surface of the tubing 479 and an inner surface of the connector 478. In some examples, the pressure sensor 489 may be positioned along a recess formed in the tubing 479 (e.g., a recess formed along the outer surface of the tubing 479). In other examples, the pressure sensor 489 may be positioned along a recess formed in the wall of the connector 478.

It can be appreciated from FIG. 8 that the pressure sensor 489 may be in indirect contact with fluid passing through an inner lumen 488 of the connector 478. The flow of fluid through the connector 478 is depicted by the arrow 492. For example, FIG. 8 illustrates that the fluid may contact a portion of the tubing 479 which, in turn, contacts the pressure sensor 489 positioned between the tubing 479 and the connector 478. It can be appreciated that shielding the sensor 489 from direct contact with fluid passing through the lumen 488 may allow the pressure sensor 489 to be more easily replaced or exchanged for an alternative pressure sensor 489. Further, because the sensor 489 may not be in direct contact with fluid (e.g., blood) passing through the lumen 488, a variety of materials may be utilized to construct the pressure sensor 489. Example materials which may be utilized to construct the pressure sensor 489 are disclosed herein.

FIG. 8 further illustrates that the connector 478 may further include an electrical connection 490 (e.g., electrical wire, etc.) which may extend from the pressure sensor 489 to a sensor interface unit 490 located within the pump 416 (shown in FIG. 5). As discussed herein, the pressure sensor 482 may measure the pressure of a fluid passing through the lumen 488 of the connector 478 and convert that pressure into a voltage output. Further, the voltage output may be sent from the sensor 489 to the sensor interface unit 486 integrated within the pump 416. The voltage output may be utilized to detect potential blockages or excessive pressures in the blood flow circuit.

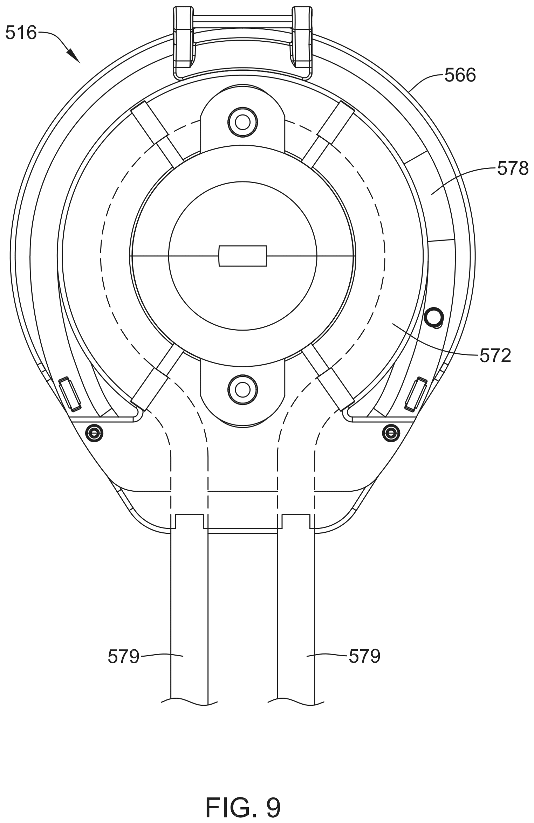

FIG. 9 illustrates a top view of an illustrative roller pump 516. The roller pump 516 may be similar in form and function to the roller pump 116 utilized with the heart lung machine 100 described with respect to FIG. 1. For example, FIG. 9 illustrates that the pump 516 may include a pump stator 566 configured to retain a pump tube 572 similar in form and function to the stator 166 and pump tube 172 described herein. For example, FIG. 9 illustrates the pump tube 572 positioned within the pump stator 566. FIG. 9 further illustrates that the roller pump 516 may include a pressure sensing region 578 integrated within the roller pump 516. For example, FIG. 9 illustrates that the pressure sensing region 578 may be positioned within the pump stator 566 and adjacent the pump tube 572. Accordingly, as will be discussed in greater detail below, one or more components of the pressure sensing region 578 may be in direct contact with the pump tube 572, having fluid passing therethrough, disposed within the roller pump 516.

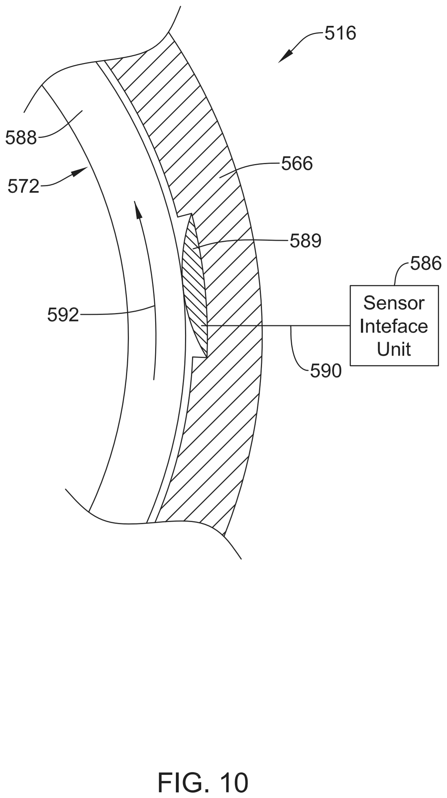

FIG. 10 illustrates the pressure sensor 589 integrated within a portion (e.g., the pressure sensing region 578) of the roller pump 516. FIG. 10 illustrates that the pressure sensor 589 may be positioned within a recess extending along the inner surface of the pump stator 566. Accordingly, as shown in FIG. 10, the pressure sensor 589 may be in direct contact with the outer surface of the pump tube 572 disposed within the roller pump 516. The flow of fluid through the lumen 588 of the pump tube 572 is depicted by the arrow 592. As discussed herein, having the pressure sensor 589 directly contact the fluid passing through the inner lumen 588 may provide higher accuracy measurements as compared with pressure sensors which are not in direct contact with the fluid passing through the inner lumen 588.

FIG. 10 further illustrates that the roller pump 516 may further include an electrical connection 590 (e.g., electrical wire, etc.) which may extend from the pressure sensor 589 to a sensor interface unit 586 located adjacent the roller pump 516. As discussed herein, the pressure sensor 589 may measure the pressure of a fluid passing through the lumen 588 of the pump tube 572 and convert that pressure into a voltage output. Further, the voltage output may be sent from the pressure sensor 589 to the sensor interface unit 586 integrated within the pump 516. The voltage output may be utilized to detect potential blockages or excessive pressures in the blood flow circuit.

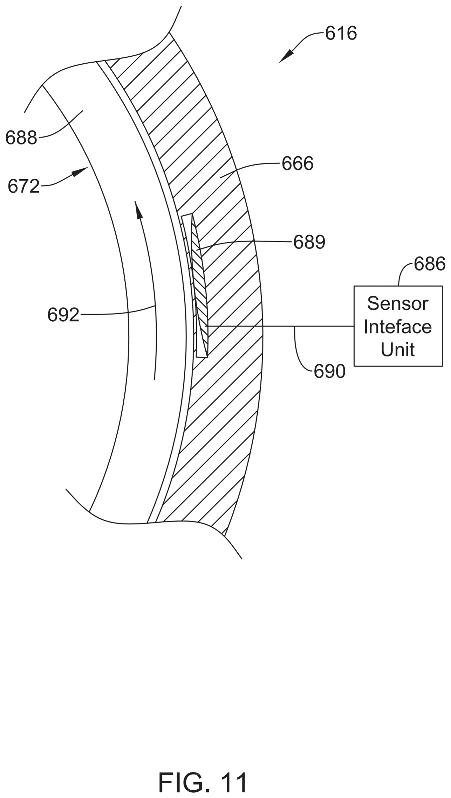

FIG. 11 illustrates another example pressure sensor 689 integrated within a portion (e.g., the pressure sensing region 578) of a roller pump 616. The roller pump 616 may be similar in form and function to the roller pump 516 described herein. FIG. 11 illustrates an example in which a pressure sensor 689 may be integrated within a cavity formed within the wall of the stator 666. In an alternative embodiment (not shown in the Figures), the pressure sensor 689 may be positioned along a recess, cavity, etc. formed in the pump tube 672 (e.g., a recess formed along the outer surface of the pump tube 672).

It can be appreciated from FIG. 11 that the pressure sensor 689 may be in indirect contact with the pump tube 672. For example, FIG. 11 illustrates that fluid flowing through the pump tube 672 (the flow of fluid within the lumen 688 of the pump tube 672 is depicted by the arrow 692) may contact the pump tube 672, whereby an outer surface of the pump tube 672 may contact a portion of the stator 666 which, in turn, contacts the pressure sensor 689 (which is positioned within a cavity formed within the wall of the stator 666). It can be appreciated that shielding the sensor 689 from direct contact with fluid passing through the lumen 688 may allow the pressure sensor 689 to be more easily replaced or exchanged for an alternative pressure sensor 689. Further, because the sensor 689 may not be in direct contact with fluid (e.g., blood) passing through the lumen 688, a variety of materials may be utilized to construct the pressure sensor 689. Example materials which may be utilized to construct the pressure sensor 689 are disclosed herein.

FIG. 11 further illustrates that the roller pump 616 may further include an electrical connection 690 (e.g., electrical wire, etc.) which may extend from the pressure sensor 689 to a sensor interface unit 686 located adjacent the roller pump 616. As discussed herein, the pressure sensor 689 may measure the pressure of a fluid passing through the lumen 688 of the pump tube 672 and convert that pressure into a voltage output. Further, the voltage output may be sent from the pressure sensor 689 to the sensor interface unit 686 integrated within the pump 616. The voltage output may be utilized to detect potential blockages or excessive pressures in the blood flow circuit.

The materials that can be used for the various components of the system 100 (and/or other systems disclosed herein) may include those commonly associated with medical device systems and components of the system 100 disclosed herein.

The components of the system 100 may be made from a metal, metal alloy, polymer (some examples of which are disclosed below), a metal-polymer composite, ceramics, combinations thereof, and the like, or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), high-density polyethylene, low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as VESTAMID^{®}, GRILAMID^{®} available from EMS American Grilon, and/or the like), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY^{®}, PHYNOX^{®}, and the like); platinum enriched stainless steel; titanium; combinations thereof; and the like; or any other suitable material.

In at least some embodiments, portions or all of the system 100 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of the system 100 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the system 100 to achieve the same result.

In some embodiments, a degree of Magnetic Resonance Imaging (MRI) compatibility is imparted into the system 100. For example, the system 100, or portions thereof, may be made of a material that does not substantially distort the image and create substantial artifacts (e.g., gaps in the image). Certain ferromagnetic materials, for example, may not be suitable because they may create artifacts in an MRI image. The system 100, or portions thereof, may also be made from a material that the MRI machine can image. Some materials that exhibit these characteristics include, for example, tungsten, cobalt-chromium-molybdenum alloys (e.g., UNS: R30003 such as ELGILOY^{®}, PHYNOX^{®}, and the like), nickel-cobalt-chromium-molybdenum alloys (e.g., UNS: R30035 such as MP35-N^{®} and the like), nitinol, and the like, and others.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. A heart lung machine, comprising:
a roller pump configured to pump blood through a blood circuit;
a first pump tube engaged with a portion of the roller pump;
a first connector having a first end and a second end, wherein the first end of the first connector is coupled to a first end of the pump tube;
a first pressure sensor coupled to the first connector or the roller pump; and
a sensor interface unit coupled to the roller pump, wherein the sensor interface unit is in communication with the first pressure sensor.

2. The heart lung machine of claim 1, wherein the first pressure sensor is attached to the first connector, and the second end of the first connector is attached to a fluid tube of the blood circuit.

3. The heart lung machine of claim 2, wherein the first connector includes an inner surface defining a lumen, and wherein the first pressure sensor is configured to be positioned within a recess extending along the inner surface of the first connector.

4. The heart lung machine of claim 3, wherein the first pressure sensor is configured to directly contact fluid flowing through the lumen.

5. The heart lung machine of claim 4, further comprising an electrical connection extending between the first pressure sensor and the sensor interface unit.

6. The heart lung machine of claim 5, wherein the first end of the first connector is releasably attached to a retention member of the pump and extends through a wall of the retention member of the pump.

7. The heart lung machine of claim 6, wherein a second end of the pump tube is coupled to a first end of a second connector, wherein the first end of the second connector extends through a wall of the retention member of the pump, and wherein the entire pump tube is positioned within the pump.

8. The heart lung machine of claim 2, wherein a first end of the fluid tube is configured to extend into a lumen defined by an inner surface of the first connector, and wherein the first pressure sensor is configured to be positioned between an outer surface of the fluid tube and the inner surface of the first connector.

9. The heart lung machine of claim 8, wherein the first pressure sensor is configured to be positioned within a recess extending along the outer surface of the fluid tube.

10. The heart lung machine of claim 9, wherein a wall of the fluid tube separates the first pressure sensor from a fluid flowing through a lumen of the fluid tube, wherein the wall of the fluid tube is configured to engage the first pressure sensor in response to a change in a pressure of the fluid flowing through the lumen of the fluid tube.

11. A heart lung machine, comprising:
a roller pump configured to pump blood through a blood circuit;
a connector assembly coupled to the roller pump, comprising:
a connector having a first end region, a second end region and an inner surface; and
a pressure sensor attached to the inner surface;
wherein the first end of the connector is releasably engaged to the roller pump;
wherein the second end of the connector is engaged to a fluid tube of the blood circuit.

12. The heart lung machine of claim 11, wherein the pressure sensor is configured to be positioned within a recess extending along the inner surface of the connector, wherein the pressure sensor is configured to directly contact fluid flowing through a lumen defined by the inner surface of the connector.

13. The heart lung machine of claim 12, further comprising an electrical connection extending between the pressure sensor and a sensor interface unit positioned within the roller pump.

14. The heart lung machine of claim 11, wherein a first end of the fluid tube is configured to extend into a lumen defined by the inner surface of the connector, wherein the pressure sensor is positioned between an outer surface of the fluid tube and the inner surface of the connector; and wherein the pressure sensor is configured to be positioned within a recess extending along the outer surface of the fluid tube.

15. A heart lung machine, comprising:
a roller pump configured to pump blood through a blood circuit;
a first pump tube releasably engaged with a portion of the roller pump;
a first connector having a first end, a second end and a first sensor manifold, wherein the first end of the first connector is coupled to a first end of the pump tube, wherein the second end of the first connector is coupled to a fluid tube of the blood circuit, and wherein the sensor manifold is coupled to a first pressure sensor;
a second connector having a first end, a second end and a second sensor manifold, wherein the first end of the second connector is coupled to a second end of the pump tube, wherein the second end of the second connector is coupled to a fluid tube of the blood circuit, and wherein the second sensor manifold is coupled to a second pressure sensor; and
a sensor interface unit positioned within the roller pump;
wherein the first pressure sensor, the second pressure sensor or both the first and the second pressure sensor are directly attached to the roller pump;
wherein the sensor interface unit is in communication with the first pressure sensor and the second pressure sensor.
